# EUROPEAN PATENT APPLICATION

(11) **EP 1 916 527 A1**
(43) Date of publication of application: **30.04.2008**
(21) Application number: 07021108.1
(22) Date of filing: 24.11.2004
(51) Int. Cl.: G01N 33/68, C07D 211/40, C07D 211/54, C07D 211/56, C07F 9/00, C07D 265/00, C07D 279/00, C07D 247/00

(54) **Analysis of mass spectral data in the quiet zones**

(30) Priority: 26.11.2003 US 525478 P; 24.02.2004 US 547375 P
(62) Divisional of application: 04817995.6
(71) Applicant: Applera Corporation, Framingham, MA 01701 (US)
(72) Inventor: Pappin, Darryl J.C., Boxborough, MA 01719 (US)
(74) Representative: Grund, Martin

(57) **Abstract**

Embodiments of this invention relate to the analysis of mass spectral data in the quiet zones.

## Description

### Cross. Reference to Related Applications:

This application claims the benefit of U.S. Provisional Application No. 60/547,375, filed on November 26, 2003, incorporated herein by reference.

### Field of the Invention:

Embodiments of the present invention relate to the analysis of mass spectral data.

### Introduction:

In some embodiments, the invention pertains to methods, systems and/or compositions useful for the analysis of labels and/or labeled analytes in quiet zones of a mass spectrum. The methods, systems and/or compositions can utilize labeling reagents that can be used to produce the labeled analytes. In some embodiments, the labeling reagents can be isotopically enriched. Because the labeling reagents can be isotopically enriched, label fragment ions generated by fragmentation of a label in a mass spectrometer can, in some embodiments, produce an isotopic cluster of distinct peak configuration. Analysis of the labels, or fragment ions thereof, can, in some embodiments, be used to determine the identity and/or quantity of an analyte or analytes in one or more samples.

In accordance with some embodiments of the present invention, the labeling reagents that fragment to produce the isotopic clusters observed in the mass spectrum can be directed to "quiet zones" across a mass spectrum. The "quite zones" can depend on the type of analyte to be determined. For example, the quiet zones can be determined by measuring intensity information for a large number of spectra of an analyte type, such as peptides, summing the intensity information and determining the "quiet zones" from the summed result. The "quiet zones" are areas where little or no mass intensity information exists in the summed result for the analyte type or types. By directing the analysis to the quiet zones, where few or no analyte fragment ions are detected, it is possible to improve the reliability of any qualitative and/or quantitative analysis of the label based on determination of the label fragment ions.

### Definitions:

### For the purposes of interpreting of this specification, the following definitions will apply and whenever appropriate, terms used in the singular will also include the plural and vice versa:

As used herein, "analyte" refers to a molecule of interest that may be determined. Non-limiting examples of analytes include, but are not limited to, proteins, peptides, peptide nucleic acids (PNA), nucleic acids (both DNA or RNA), carbohydrates, lipids and other small molecules with a molecular weight of less than 1500 Daltons (Da). The source of the analyte, or the sample comprising the analyte, is not a limitation as it can come from any source. The analyte or analytes can be natural or synthetic. Non-limiting examples of sources for the analyte, or the sample comprising the analyte, include cells or tissues, or cultures (or subcultures) thereof. Other non-limiting examples of analyte sources include, but are not limited to, crude or processed cell lysates, body fluids, tissue extracts, cell extracts or fractions (or portions) from a separations process such as a chromatographic separation, a 1D electrophoretic separation, a 2D electrophoretic separation or a capillary electrophoretic separation. Body fluids include, but are not limited to, saliva, blood, urine, feces, spinal fluid, cerebral fluid, amniotic fluid, lymph fluid or a fluid from a glandular secretion. By processed cell lysate we mean that the cell lysate is treated, in addition to the treatments needed to lyse the cell, to thereby perform additional processing of the collected material. For example, the sample can be a cell lysate comprising one or more analytes that are peptides formed by treatment of the cell lysate with a proteolytic enzyme to thereby digest precursor peptide or protein.

As used herein, "fragmentation" refers to the breaking of a covalent bond.

As used herein, "fragment" refers to a product of fragmentation (*noun*) or the operation of causing fragmentation (*verb*).

As used herein an "isotopic cluster" refers to a grouping of intensity peaks in a mass spectrum that are associated with a single compound (e.g. a label). The compound or label can be isotopically enriched. The isotopic cluster can include a single main isotope peak and two or more side peaks. The side peaks can be of lower intensity than the main isotope peak, and can be both down-mass and up-mass of the main peak. Although the separation between the main peak and side peaks can be measured in whole numbers, for example, 1, 2, 3, etc. Da, the separation may also be measured as non-whole numbers, for example, 0.5, 1.2, etc. For example, an isotopic cluster with a main peak at X Da can include the intensity contribution of at least one up-mass side peak at *X* + *1* Da and the intensity contribution of at least one down-mass side peak at *X* - *1* Da.

As used herein, "isotopically enriched" refers to a compound (e.g. label, labeling reagent or label fragment ion) that has been enriched with one or more high mass or "heavy" isotopes (e.g. stable isotopes such as Deuterium, ¹³C, ¹⁵N, ¹⁸O, ³⁷Cl or ⁸¹Br). By "enriched", we mean the application of processes that introduce high mass isotopes into a compound in excess of the natural isotopic abundance. When a compound or fragment comprises at least one high mass isotope, we sometimes refer to the compound or fragment as being "heavy". Because isotopic enrichment is not 100% effective, there can be impurities of the compound that are of lesser states of enrichment and these will have a lower mass. Likewise, because of over-enrichment (undesired enrichment) and because of natural isotopic abundance, there can be impurities of greater mass. This is why a sample of a single isotopically enriched compound (or part thereof) can, when subjected to analysis in a mass spectrometer, produce an isotopic cluster of fragment ions having both at least one up-mass side peak and at least one down-mass side peak in addition to the main peak attributable to the majority of the compound.

As used herein, "natural isotopic abundance" refers to the level (or distribution) of one or more isotopes found in a compound based upon the natural prevalence of an isotope or isotopes in nature. For example, a natural compound obtained from living plant matter will typically contain about 1.08 % ¹³C relative to ¹²C.

As used herein, "label" refers to a moiety suitable to mark an analyte for determination. The term label is synonymous with the terms tag and mark and other equivalent terms and phrases. For example, a labeled analyte can be referred to as a tagged analyte or a marked analyte. Labels can be used in solution or can be used in combination with a solid support. Accordingly, a labeling reagent or labeled analyte can exist in solution or be deposited on, or linked to, a solid support.

As used herein, "label fragment ion" refers to an ion comprising at least part of a label. The "label fragment ion" can be produced by fragmentation of the label in a mass spectrometer. The label can fragment, in a mass spectrometer, whether or not it is linked to an analyte. The label fragment ion is typically not an ion produced by fragmentation of the analyte.

As used herein, "support", "solid support" or "solid carrier" refers to any solid phase material. A labeling reagent can be immobilized to a support and thereafter used to label one or more analytes. An analyte can be immobilized to a support and then labeled with a labeling reagent. A labeled analyte can be immobilized to a support for processing. Solid support encompasses terms such as "resin", "synthesis support", "solid phase", "surface" "membrane" and/or "support". A solid support may be composed of organic polymers such as polystyrene, polyethylene, polypropylene, polyfluoroethylene, polyethyleneoxy, polyvinylidene difluoride (PVDF) and polyacrylamide, as well as copolymers and grafts thereof. A solid support may also be inorganic, such as glass, silica, controlled-pore-glass (CPG), or reverse-phase silica. The configuration of a solid support may be in the form of beads, spheres, particles, granules, a gel, a membrane or a surface. Surfaces may be planar, substantially planar, or non-planar. Solid supports may be porous or non-porous, and may have swelling or non-swelling characteristics. A solid support may be configured in the form of a well, depression or other container, vessel, feature or location. A plurality of solid supports may be configured in an array at various locations, addressable for robotic delivery of reagents, or by detection methods and/or systems.

### Description Of Various Embodiments Of The Invention:

In some embodiments, this invention pertains to methods, systems and/or compositions useful for the analysis of labels and/or labeled analytes in quiet zones of a mass spectrum. By directing the analysis to quiet zones, it is possible to maximize the dynamic range of the analysis. This can be useful for qualitative and/or quantitative analysis of analytes.

In some embodiments, a convoluted spectrum can be compiled from output data obtained from an analyzer such as a mass spectrometer. The output data can be used to construct a convoluted spectrum for one or more fragment ions produced by the analysis of a sample. The convoluted spectrum can be deconvoluted. Deconvolution can provide normalized peak intensity information for the fragment ions of one or more isotopic clusters. The isotopic clusters can be directed to the quiet zones of the mass spectrum. Because the normalized peak intensity for the isotopic cluster can be determined, and because the intensity of peaks of the isotopic cluster can define a particular label, the normalized peak intensity can be used for both qualitative and/or quantitative determinations of the fragment ions of one or more labels (i.e. a label fragment ion). Where the presence and/or amount of label fragment ions of a label can be correlated with the presence and/or amount of an analyte or analytes in one or more samples subjected to analysis by the analyzer, the presence and/or amount of analyte or analytes in one or more samples can be determined by the qualitative and/or quantitative analysis of the one or more label fragment ions and their associated isotopic clusters. Exemplary methods for such deconvolution of spectra can be found in copending and co-owned US Patent Application Serial No. 10/916,629 filed on August 12, 2004, incorporated herein by reference.

In some embodiments of the present invention, the convoluted spectrum defines a spectral region of interest where isotopic clusters can be generated from the fragmentation of labeling reagents, whether or not linked to an analyte. In some embodiments, the labeling reagents can be isotopically enriched. In some embodiments, the isotopically enriched labeling reagents can be isobaric and/or isomeric.

Fragmentation of a labeled analyte can produce fragment ions for the analyte, for the label or for a portion (fragment) of the label. If the label is isotopically enriched, depending on the point of fragmentation, the one or more heavy atoms of the isotopically enriched label may or may not exist in the label fragment ion. When more than one heavy isotope is incorporated into a label, it is possible that some of the heavy isotopes remain with the label fragment ion observed in the mass spectrum whilst others remain in fragments that are not observed in the mass spectrum because they lack charge (e.g. thru neutral loss). In some embodiments, the label fragment ion comprising at least one heavy isotope is the predominate ion of an isotopic cluster observed in the mass spectrum.

Fragmentation of the labeling reagents (or the labeled analytes) can occur by subjecting the label and/or the labeled analyte to dissociative energy (e.g. collision-induced dissociation (CID)). The normalized peak intensity for the label fragment ions that define each isotopic cluster can correlate with the presence and/or quantity of label that produces the isotopic cluster. Information for each isotopic cluster can correlate with the presence and/or quantity of an analyte. The various isotopic clusters that define the convoluted spectrum can each be attributable to a different label or a different labeled analyte.
Fragment ions of the labels or labeled analytes can be obtained from the same or from different samples. In some embodiments, fragment ions of the labels or labeled analytes can be obtained from different samples that are combined into one sample that is subjected to analysis by the analyzer. Accordingly, the analysis of the convoluted spectrum can be used in the qualitative and/or quantitative analysis of one or more analytes in one or more samples.

In some embodiments, MSⁿ analysis is performed in a tandem mass spectrometer where n is greater than or equal to 2. For analysis where n is greater than or equal to 2, ions can be selected from an initial MS analysis and directed to a separate MS analyzer for subsequent analysis. The selected ions can be subjected to dissociative energy (to induce fragmentation) before being directed to a second MS analyzer. By selecting specific ions (of labels or labeled analytes) for subsequent analysis, the complexity of the sample can be reduced so that random fragments for other analytes not of interest in the sample can be eliminated from the mass analysis. In this way, only fragments of the selected ion or ions are detected in the second, or subsequent, mass analyzer. If the analyte is, for example, a labeled peptide and it is subjected to fragmentation, only fragments of the label and the peptide will be observed in the spectrum of the mass analysis of the selected ions in the subsequence mass analysis. Where the labels are directed to the quiet zones of the mass spectrum for peptide analysis, only label fragments ions should be observed in the quiet zones since the possible fragment ions for peptides lie outside these zones.

It is not a requirement of this invention that analysis be performed with a tandem mass spectrometer. Moreover, the mass spectrometer can be run in either positive or negative ion mode. Additional mass spectrometry instruments and fragmentation methods that can be used in combination with embodiments of this invention include post-source decay in MALDI-MS instruments and high-energy CID using MALDI-TOF (time of flight)-TOF MS. For a recent review of tandem mass spectrometers please see: R. Aebersold and D. Goodlett, Mass Spectrometry in Proteomics. Chem. Rev. 101: 269-295 (2001). Also see United States Patent No. 6,319,476, herein incorporated by reference, for a discussion of TOF-TOF mass analysis techniques.

The location of "quiet zones" in a spectrum can depend on the nature of the sample or the analyte being analyzed. Fragmentation in a mass spectrometer is not entirely random and indeed is fairly reproducible for analytes of a particular genus (e.g. peptides or proteins). Though many different types of fragment ions can be generated in a mass spectrometer, the weakest bonds will typically be the first to fragment. For example, if the sample to be analyzed primarily comprises peptides and/or proteins, the fragment ions observed in a mass spectrum of the sample will primarily comprise similar fragment types (e.g. ions of shorter peptides, amino acids and parts of peptides and amino acids). Accordingly, reproducible fragmentation of the peptides and proteins can occur such that there are areas of the spectrum where few or no fragment ions are detected because fragmentation of a peptide or protein simply does not produce detectable ions of these certain mass to charge ratios (e.g. the signals can be weak or not detectable). These areas of the mass to charge (m/z) spectrum are the "quiet zones" when the analyte is a peptide and/or protein. Quiet zones for nucleic acids would typically differ from those observed for proteins and peptides since the products of fragmenting a nucleic acid in a mass spectrum will differ from those obtained from fragmentation of a peptide. However, because of the consistency of fragmentation for analytes of a particular genus, the quiet zones are typically reproducible and analyte dependent.

One way to determine the quiet zones for the analysis of a particular analyte is to sum mass spectra obtained for the analysis of fragment ions of the analyte genus and then determine the quiet zones (i.e. areas where little or no fragment ions are observed in the summed result). The spectra can be randomly selected spectra for the analyte of interest. The spectra to be summed can be presently collected or they can be historical (e.g. supplied by a database). Accordingly, the source of the spectra to be summed is not a limitation.

Once the quiet zones are established, labeling reagents that produce label fragment ions in the quiet zones can be selected based upon knowledge of the fragmentation patterns of compounds. For isotopically enriched labels, the possible isotopes and their distribution within the labeling reagent should be considered so that the label fragment ions are directed to the quiet zones. For example, sets of isobaric and/or isomeric labeling reagents can be prepared such that within the set, all of the labels produce a label fragment ion within one or more of the quiet zones but wherein each label of the set comprises a different distribution of isotopes such that each different label produces a unique label fragment ion of a unique mass to charge ratio within the quiet zone. In this way, each label of the set can be independently determined and/or quantitated.

With reference to Figure 1 and Example 1, a composite of the summed intensity data for approximately 75,000 random historical spectra of peptide/protein analysis is presented. Expansion plots of the 0-75 atomic mass units (amu) (Figure 2), 76-150 amu (Figure 3) and 151-225 (amu) (Figure 4) are also presented. The quiet zones can be determined by visual inspection or by programmed analysis of the composite spectrum or the expansion plots. Table 1, lists the "quiet zones" obtained by visual analysis of the composite spectrum or the expansion plots. "Quiet zones" comprise the area of the mass spectrum where little or no peak intensity is observed in the summed intensity data.

**Table 1: Potential "Quiet Zones" For Selection Of Label Fragment Ion m/z For Peptides and Proteins**

| **M/z start - end** |
|---|
| |
| 10-14 |
| 19-22 |
| 24-26 |
| 31-38 |
| 40-40 |
| 46-50 |
| 52-52 |
| 58-58 |
| 61-69 |
| 71-71 |
| 74-83 |
| 89-97 |
| 103-109 |
| 113-119 |
| 121-125 |
| 128-128 |
| 131-135 |
| 137-147 |
| 149-154 |
| 156-156 |
| 160-174 |
| 177-182 |
| 184-184 |
| 188-189 |
| 191-191 |
| 202-207 |
| 210-210 |
| 216-222 |
| 224-226 |

Accordingly, in some embodiments, this invention pertains to a method comprising summing the intensity of a representative number of fragmentation spectra obtained for a selected analyte to thereby obtain a composite spectrum. By "fragmentation spectra" we mean spectra obtained after the analyte has been subjected to dissociative energy sufficient to fragment at least a portion of the molecules of the analyte in the sample. By representative number we mean a sufficient number of spectra that provides intensity data for a large set of possible fragments that are typically observed for the selected analyte. For example, at least 1,000 mass spectra of the analyte of interest can be summed. The quiet zones of the composite spectrum can then be determined from analysis of the composite spectrum.

The confidence of the quiet zones determination can depend on the number of spectra used to generate the summed result. The greater the number of spectra used to generate the summed result, the higher the confidence will be that there will be little or no interference in a designated quiet zone. In some embodiments, the intensity of at least 5,000 spectra can be summed. In some embodiments, the intensity of at least 10,000 spectra can be summed. In some embodiments, the intensity of at least 25,000 spectra can be summed. In some embodiments, the intensity of at least 50,000 spectra can be summed. In some embodiments, it is possible, or desirable, to sum even more spectra. In some embodiments, the spectra to be summed are randomly selected. The spectra to be summed can be presently collected or they can be historical (e.g. supplied by the archives of a database).

In some embodiments, the method can further comprise selecting one or more labeling reagents that, when used to label the selected analyte, will fragment in a mass spectrometer to produce at least one label fragment ion having a mass to charge ratio located in one of the quiet zones of the composite spectrum: By choosing a labeling reagent that fragments to produce a label fragment ion directed to the quiet zone, the analysis, and particularly the quantitation, of the label fragment ion can be highly reliable. In some embodiments, two or more isobaric or isomeric labeling reagents can be used to label the same analyte, wherein the labeling reagents fragment to produce fragment ions that differ by one atomic mass unit (i.e. a Dalton) wherein a fragment from each of the different labeling reagents possesses a mass to charge ratio located in one of the quiet zones for the analyte of interest. In some embodiments, the main peak of each of two isotopic clusters can be separated by a single Dalton.

In some embodiments, the selected labeling reagent can be isotopically enriched. The isotopically enriched labeling reagent can be used to label an analyte. In some embodiments of using the isotopically enriched labeling reagent, the method can further comprise labeling a peptide or protein (or a sample comprising a peptide or protein) with the selected labeling reagent. In some embodiments of using the isotopically enriched labeling reagent, the method can further comprise labeling a nucleic acid (or a sample comprising a nucleic acid) with the selected labeling reagent. Regardless of the nature of the analyte, the method can further comprise detecting at least one label fragment ion, produced by fragmentation of the labeled analyte in a mass spectrometer, in a quiet zone of the analyte type selected. Table 1 lists quiet zones for the analysis of analytes that are peptides or proteins.

In some embodiments, the designated "quiet zones" can be determined for a composite of two or more different analyte types. For example, if a sample comprises peptides and nucleic acids, a composite of the intensity spectra and/or tabular data for both fragmented peptides and fragmented nucleic acids can be prepared and analyzed to thereby determine the "quiet zones" for the composite of the two analyte or more types. It should be self-evident that the method, system and/or composition embodiments of this invention can be applied to more than one selected analyte in this manner.

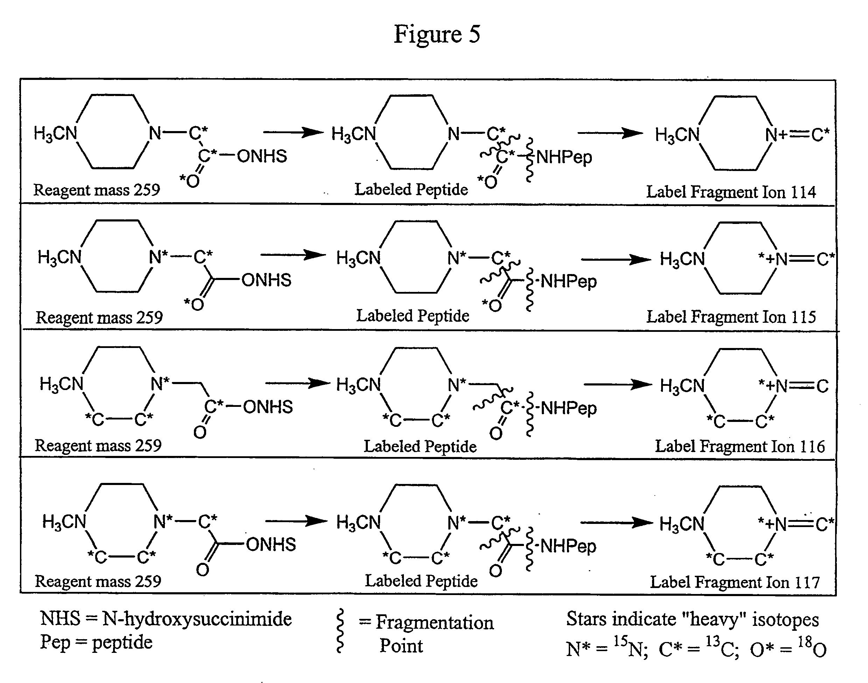

In some embodiments, the labeling reagent can be a small molecule that produces a label fragment ion having a mass to charge ratio of less than 250 amu. For example, the label that produces the label fragment ion can be from a piperidine compound, a piperazine compound or a morpholine compound. For example, the labeled analyte can be a compound of the formula: 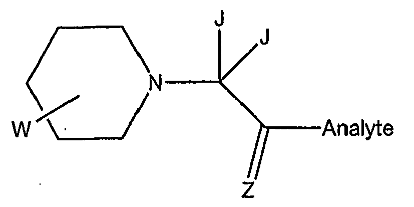 wherein: Z is O, S, NH or NR¹; each J is the same or different and is H, deuterium (D), R¹, OR¹, SR¹, NHR¹, N(R¹)₂, fluorine, chlorine, bromine or iodine; W is an atom or group that is located ortho, meta or para to the ring nitrogen and is NH, N-R¹, N-R², P-R¹, P-R², O or S; each carbon of the heterocyclic ring has the formula CJ₂; each R¹ is the same or different and is an alkyl group comprising one to eight carbon atoms which may optionally contain a heteroatom or a substituted or unsubstituted aryl group wherein the carbon atoms of the alkyl and aryl groups independently comprise linked hydrogen, deuterium and/or fluorine atoms; and R² is an amino alkyl, hydroxy alkyl, thio alkyl group or a cleavable linker that cleavably links the reagent to a solid support wherein the amino alkyl, hydroxy alkyl or thio alkyl group comprises one to eight carbon atoms, which may optionally contain a heteroatom or a substituted or unsubstituted aryl group, and wherein the carbon atoms of the alkyl and aryl groups independently comprise linked hydrogen, deuterium and/or fluorine atoms. Some embodiments of a useful piperazine labeling reagent for peptide analysis can produce label fragment ions in the quiet zone of 113-119 amu (See: Figure 5). The labeling reagents can be isotopically enriched. The labeling reagents can be isomeric or isobaric. Other non-limiting examples of suitable labeling reagents, including sets of isomeric and/or isobaric labeling reagents can be found in co-pending and commonly owned international patent application no. WO2004/070352, incorporated herein by reference.

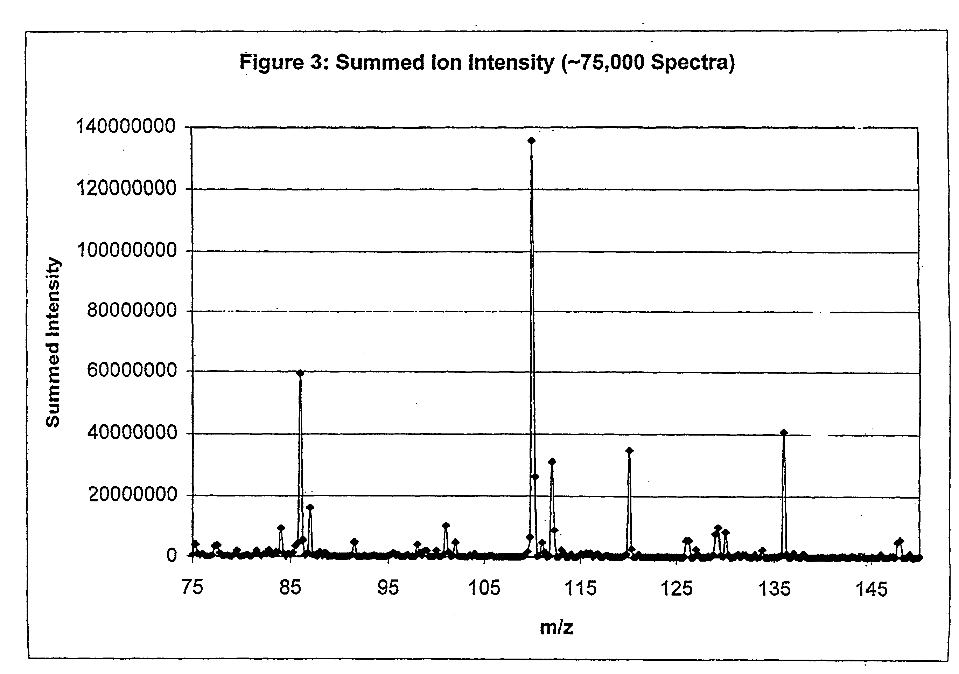

Figure 5 is an illustration of a set of four piperazine based isotopically enriched isobaric labeling reagents that can be used to label an analyte, such as a peptide or protein. Because they are isobaric, they comprise the same structure and the same mass, albeit with a unique distribution of heavy isotopes. As the illustration shows, these four labeling reagents fragment identically. However, because of the unique distribution of heavy isotopes within each of the labeling reagents, each of the four labeling reagent produces a label fragment ion of unique mass to charge ratio within the set. Specifically, the mass to charge ratio of the four label fragment ions is separated by one Dalton in the range from 114-117 m/z. Accordingly, these label fragment ions are directed to the quiet zone of 112-119 m/z for peptide/protein analysis as observed in Figure 3 and identified in Table 1.

In some embodiments, this invention pertains to a method comprising detecting, in a mass spectrometer, a label fragment ion produced by fragmentation of a labeled analyte in the mass spectrometer wherein the label fragment ion has a mass to charge ratio in a quiet zone for the selected analyte. The label of the labeled analyte can be isotopically enriched. The label that produces the label fragment ion can be isomeric or isobaric. The labeled analyte can be any analyte.

In some embodiments, this invention pertains to a label fragment ion, produced by fragmentation of a labeled analyte in a mass spectrometer, having a mass to charge ratio in a quiet zone for the selected analyte. The label that produces the label fragment ion can be isotopically enriched. In some embodiments, the label fragment ion can comprise two different heavy isotopes (e.g. at least one ¹³C and at least one ¹⁵N). The label that produces the label fragment ion can be isomeric or isobaric. The labeled analyte can be any analyte.

In some embodiments, this invention pertains to a label fragment ion, produced by fragmentation of a labeled biomolecule (e.g. a peptide, protein, carbohydrate, nucleic acid (DNA or RNA), lipid, etc.) in a mass spectrometer, having a mass to charge ratio in a region of the mass spectrum where expected fragments of the biomolecule are of low intensity or are absent. For example, expected fragments of a peptide include amino acids and shorter peptides and fragments thereof. The label that produces the label fragment ion can be isotopically enriched. The label that produces the label fragment ion can be isomeric or isobaric. The labeled analyte can be any analyte.

In some embodiments, this invention pertains to a label fragment ion, produced by fragmentation of a labeled biomolecule in a mass spectrometer, having a mass to charge ratio in a zone of the mass spectrum where expected fragments of the biomolecule are of low intensity or are absent. The label that produces the label fragment ion can be isotopically enriched. In some embodiments, the label fragment ion can comprise two different heavy isotopes (e.g. at least one ¹³C and at least one ¹⁵N). The label that produces the label fragment ion can be isomeric or isobaric. The labeled analyte can be any analyte.

In still some embodiments, this invention pertains to mass analyzers (e.g. mass spectrometers) in combination with labeled analytes that generate the labeled fragment ions as hereinbefore described. In still some other embodiments, this invention pertains to mass analyzers (e.g. mass spectrometers) in combination with the operation of any of the herein before described methods.

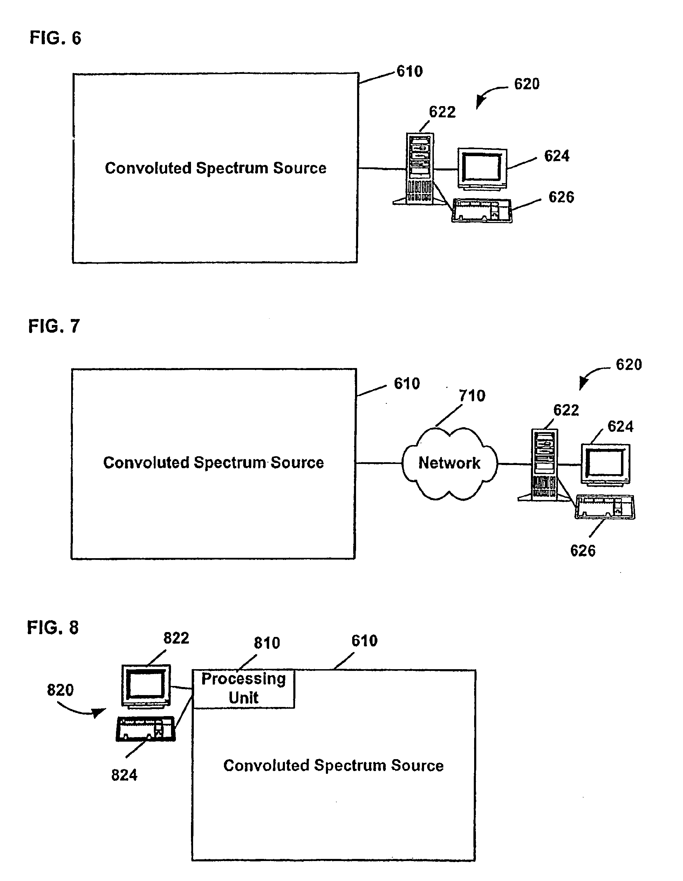

FIG. 6 is a block diagram of a system in which some embodiments of the present invention can be practiced. In FIG. 6, a convoluted spectrum source 610 can be coupled to a computer system 620. Convoluted spectrum source 610 can include, but not be limited to, for example, a mass spectrometer (MS), including those capable of post-source decay, a MS/MS, an MSⁿ, a quadropole MS, as well as data files from historical MS analyses. Computer system 620 can include a processing unit 622 coupled to a display 624 and an input device 626, for example, a keyboard. Other input devices 626 can include, but are not limited to, an electronic writing tablet, a mouse, a voice activated input device, etc. Processing unit 622 can include a processor, for example, a microprocessor or multiple processors, coupled to a memory and a mass storage device. For example, while in no way intended to limit the possible configurations of processing unit 622, the processor can include a microprocessor, the memory can include a random access memory (RAM) and the mass storage device can include a hard disk device. Computer system 620 can receive convoluted spectrum data, historical spectra and/or known isotopic cluster information from convoluted spectrum source 610 and can de-convolute the convoluted spectrum data using the known isotopic cluster information.

FIG. 7 is a block diagram of another system in which some embodiments of the present invention can be practiced. In FIG. 7, convoluted spectrum source 610 and computer system 620 from FIG. 6 can be coupled, in FIG. 7, via a network 710, for example, a communications network, the Internet, a local area network (LAN), a wide area network (WAN) and a wireless network. The operation of the system in FIG. 7, as well as similar components, are identical to the system in FIG. 6 with the exception that communication of information from convoluted spectrum source 610 to computer system 620 can occur through the network 710.

FIG. 8 is a block diagram of yet another system in which embodiments of the present invention can be practiced. In FIG. 8, convoluted spectrum source 610 can include a processing unit 810 that can be coupled to a peripheral subsystem 820 including, for example, display device 822 and input device 824. Processing unit 810 can be configured as described above in FIG. 6 for processing unit 622. The operation of the system in FIG. 8, as well as similar components, are identical to the system in FIG. 6 with the exception that processing unit 810 is located in convoluted spectrum source 610.

Although the present invention has been disclosed in detail, it should be understood that various changes, substitutions, and alterations can be made herein. Moreover, although software and hardware are described to control certain functions, such functions can be performed using either software, hardware or a combination of software and hardware, as is well known in the art. Other examples are readily ascertainable by one skilled in the art and can be made without departing from the spirit and scope of the present invention as defined by the following claims.

### Brief Description Of The Drawings:

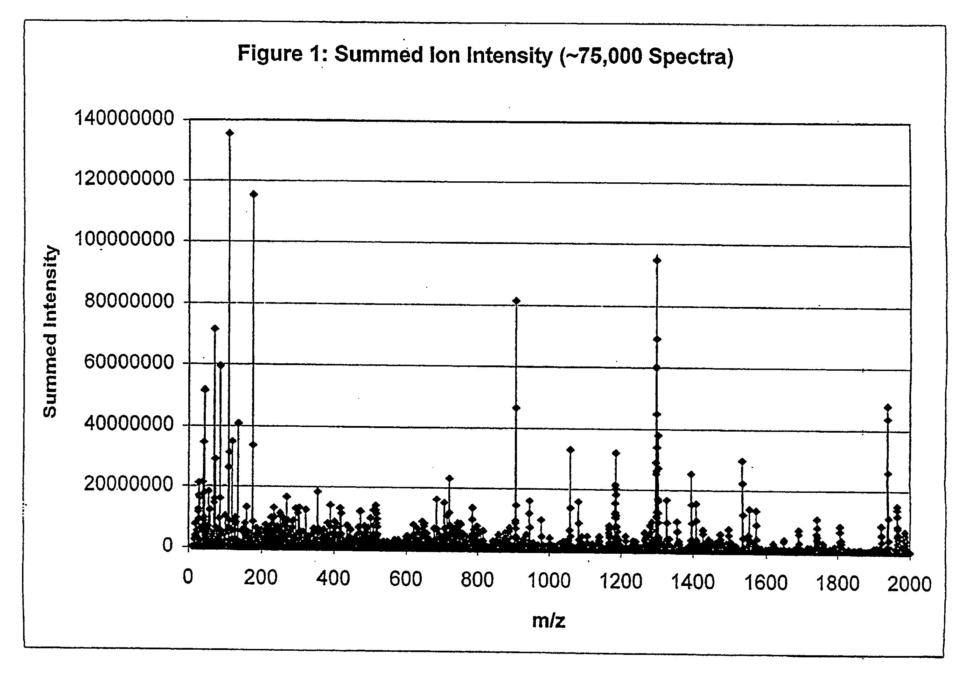

Figure 1 is a composite spectrum of the result, from 0 to 2000 atomic mass units (amu), of summed intensity data for approximately 75,000 historical collision induced dissociation (CID) mass spectra of peptide/protein containing samples obtained using an Applied Biosystems 4700 mass analyzer.

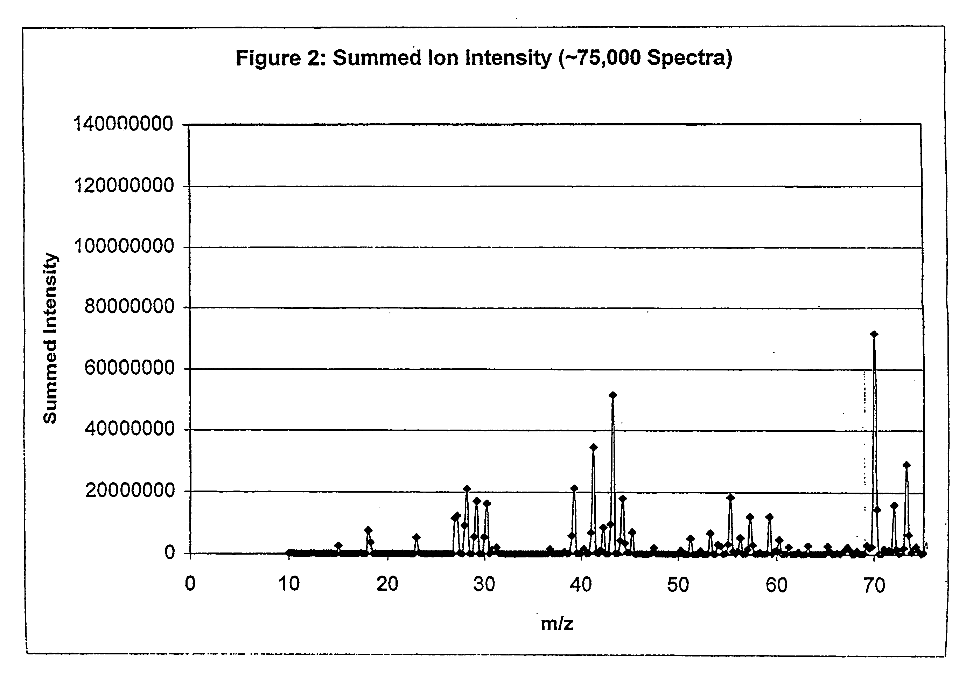

Figure 2 is an expansion plot of the composite spectrum of the result, from m/z 0 to 75 (data for 10-75), of summed intensity data for approximately 75,000 historical mass spectra of peptide/protein containing samples obtained using an Applied Biosystems 4700 mass analyzer.
Figure 3 is an expansion plot of the composite spectrum of the result, from m/z 75 to 150, of summed intensity data for approximately 75,000 historical mass spectra of peptide/protein containing samples obtained using an Applied Biosystems 4700 mass analyzer.

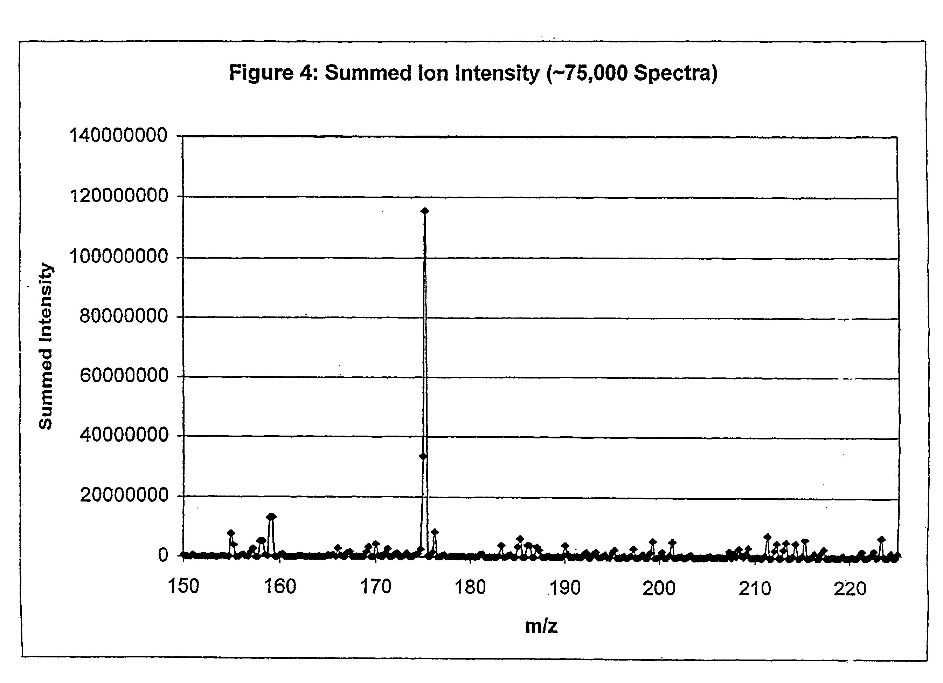

Figure 4 is an expansion plot of the composite spectrum of the result, from m/z 150 to 225, of summed intensity data for approximately 75,000 historical mass spectra of peptide/protein containing samples obtained using an Applied Biosystems 4700 mass analyzer.
Figure 5 is an illustration of the structures of a set of four isotopically enriched isobaric labeling reagents that can each fragment in a mass spectrometer to produce a label fragment ion of a different mass to charge ratio as compared with the others. The mass to charge ratio of all of the label fragment ions is in a quiet zone for peptide/protein analysis.
Figure 6 is a block diagram of a system in which embodiments of the present invention can be practiced.
Figure 7 is a block diagram of another system in which embodiments of the present invention can be practiced.
Figure 8 is a block diagram of yet another system in which embodiments of the present invention can be practiced.

### Modes For Carrying Out The Invention:

### Example 1: Determining Quiet Zones For Peptide/Protein Analysis

Figure 1 is a composite spectrum of the summed intensity of approximately 75,000 peptide CID spectra obtained using the 4700 TOF-TOF mass spectrometer from Applied Biosystems. The peptides were derived from a variety of sources, including yeast and human cell lines as well as tissue samples. The spectra are intended to provide a representation of fragment ions that have been observed across a very large population of possible amino acid sequences and compositions. These fragments would be characteristic for protein and/or peptide analysis. Close examination of the composite spectrum shows that there are regions where signal is low or absent (See: Figures 2, 3 & 4). The data for the quiet zones for peptide samples, as determined from the composite spectrum, is summarized in Table 1. For example, one of the quiet zones is from 113-119 amu. A similar composite spectrum and accompanying analysis of quiet zones can be prepared for other analytes such as nucleic acids, lipids, carbohydrates, peptide nucleic acids (PNA) and small molecules.

## Claims

1. A method comprising detecting, in a mass spectrometer, a label fragment ion produced by fragmentation of a labeled analyte in the mass spectrometer wherein the label fragment ion has a mass to charge ratio in a quiet zone for the selected analyte.

2. The method of claim 1, wherein the selected analyte is a protein, a peptide, a nucleic acid, a carbohydrate, a lipid, peptide nucleic acid or a small molecule with a molecular weight of less than 1500 Daltons (Da).

3. The method of claim 1, wherein the label fragment ion comprises one or more heavy isotopes.

4. The method of claim 3, wherein the one or more heavy isotopes are stable isotopes selected from the group consisting of deuterium, ¹³C, ¹⁵N, ¹⁸O, ³⁷Cl or ⁸¹Br.

5. The method of claim 4, wherein the label fragment ion comprises at least one ¹³C and at least one ¹⁵N.

6. The method of claim 1, wherein the label fragment ion is the predominate ion of an isotopic cluster and comprises at least one heavy isotope.

7. The method of claim 1, wherein the mass to charge ratio of the label fragment ion is from 10-14 amu, from 19-22 amu, from 24-26 amu, from 31-38 amu, from 46-50 amu, from 131-135 amu, from 137-147 amu, from 149-154 amu, from 160-174 amu, from 177-182 amu, from 188-189 amu, from 202-207 amu, from 216-222 amu or from 224-226 amu.

8. The method of claim 1, wherein the mass to charge ratio of the label fragment ion is from 61-69 amu, from 74-83 amu, from 89-97 amu, from 103-109 amu, from 113-119 amu or from 121-125 amu.

9. The method of claim 1, wherein the mass to charge ratio of the label fragment ion is 40 amu, 52 amu, 58 amu, 71 amu, 128 amu, 156 amu, 184 amu, 191 amu or 210 amu.

10. The method of claim 1, wherein the label fragment ion has a mass to charge ratio of less than 250 amu.

11. The method of claim 1, wherein the label fragment ion is not a fragment generated from the analyte.

12. The method of claim 1, wherein the label fragment ion is a fragment ion produced by fragmentation of a piperidine compound, a piperazine compound or a morpholine compound.

13. The method of claim 1, wherein the labeled analyte is a compound of the formula: 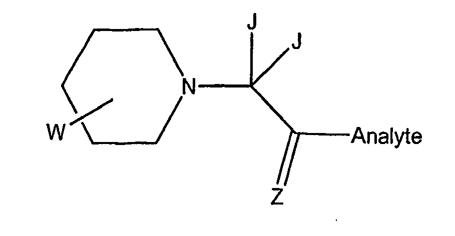 wherein:
Z is O, S, NH or NR¹;
each is the same or different and is H, deuterium (D), R¹, OR¹, SR¹, NHR¹, N(R¹)₂, fluorine, chlorine, bromine or iodine;
W is an atom or group that is located ortho, meta or para to the ring nitrogen and is NH, N-R¹, N-R², P-R¹, P-R², O or S;
each carbon of the heterocyclic ring has the formula CJ₂;
each R¹ is the same or different and is an alkyl group comprising one to eight carbon atoms which may optionally contain a heteroatom or a substituted or unsubstituted aryl group wherein the carbon atoms of the alkyl and aryl groups independently comprise linked hydrogen, deuterium and/or fluorine atoms;
and R² is an amino alkyl, hydroxy alkyl, thio alkyl group or a cleavable linker that cleavably links the reagent to a solid support wherein the amino alkyl, hydroxy alkyl or thio alkyl group comprises one to eight carbon atoms, which may optionally contain a heteroatom or a substituted or unsubstituted aryl group, and wherein the carbon atoms of the alkyl and aryl groups independently comprise linked hydrogen, deuterium and/or fluorine atoms.

14. The method of claim 13, wherein the mass to charge ratio of the label fragment ion is from 113-119 amu.

15. The method of claim 2, wherein the selected analyte is a peptide or a protein.

16. The method of claim 2, wherein the selected analyte is a nucleic acid.

17. The method of claim 1, wherein the label fragment ion is produced by subjecting, in a mass spectrometer, a selected ion of a labeled analyte to dissociative energy.

18. The method of claim 1, wherein the dissociative energy is collision induced dissociation.

19. A method comprising
a) determining one or more quiet zones in the spectrum of an analyte in a mass spectrometer;
b) selecting one or more labelling reagents that, when used to label the selected analyte, will fragment in a mass spectrometer to produce at least one label fragment ion having a mass to charge ration located in one of the quiet zones of the spectrum of the analyte.

20. A method comprising:
a) summing the intensity of a representative number of fragmentation spectra obtained for a selected analyte to thereby obtain a composite spectrum; and
b) determining one or more quiet zones in the composite spectrum.

21. A method comprising detecting, in a mass spectrometer, a label fragment ion produced by fragmentation of a labeled analyte in the mass spectrometer wherein the label fragment ion has a mass to charge ratio in a quiet zone determined for a composite of two or more selected analytes.
